# EUROPÄISCHE PATENTSCHRIFT

(11) **EP 2 411 374 B1**
(45) Veröffentlichungstag und Bekanntmachung des Hinweises auf die Patenterteilung: **19.11.2014**
(21) Anmeldenummer: 10707216.7
(22) Anmeldetag: 26.02.2010
(51) Int. Cl.: C07D 251/10

(54) **PROZESS ZUR ENANTIOMERENTRENNUNG VON 3,6- DIHYDRO-1,3,5- TRIAZINDERIVATEN**
PROCESS FOR SEPARATING ENANTIOMERS OF 3,6-DIHYDRO-1,3,5-TRIAZINE DERIVATIVES
PROCÉDÉ DE SÉPARATION ÉNANTIOMÉRIQUE DE DÉRIVÉS DE 3,6-DIHYDRO-1,3,5-TRIAZINE

(30) Priorität: 25.03.2009 DE 102009014898
(43) Veröffentlichungstag der Anmeldung: 01.02.2012
(73) Patentinhaber: Merck Patent GmbH, 64293 Darmstadt (DE)
(72) Erfinder: HELMREICH, Matthias, 64285 Darmstadt (DE); NIESERT, Claus-Peter, 64342 Seeheim-Jugenheim (DE); SCHULTE, Michael, 65474 Bischofsheim (DE); LINDNER, Wolfgang, A-3400 Klosterneuburg (AT); LAEMMERHOFER, Michael, 72070 Tübingen (DE); HOFFMANN, Christian, 95445 Bayreuth (DE)
(86) Internationale Anmeldenummer: PCT/EP2010/001219
(87) Internationale Veröffentlichungsnummer: WO 2010/108583

(56) Entgegenhaltungen:
- WO-A1-03/068397
- WO-A2-2004/089917

## Beschreibung

Die Erfindung betrifft ein Verfahren zur chromatographischen Trennung von razemischen und nicht-razemischen Gemischen der Enantiomere der Verbindungen der Formel I 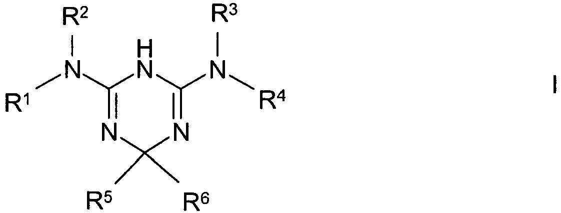 worin
- R¹, R²: jeweils unabhängig voneinander H oder A,
- R³, R⁴: jeweils unabhängig voneinander H, A, Alkenyl mit 2-10 C-Atomen, Alkinyl mit 2-10 C-Atomen, Ar oder Het,
- R⁵ und R⁶: zusammen auch Alkylen mit 2, 3, 4 oder 5 C-Atomen,
- R⁵, R⁶: jeweils unabhängig voneinander H, A, (CH₂)ₙAr, (CH₂)ₘOAr, (CH₂)ₘOA oder (CH₂)ₘOH,
- R⁵ und R⁶: zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
- Ar: unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
- A: unverzweigtes oder verzweigtes Alkyl mit 1-10 C-Atomen, worin 1-7 H-Atome durch F ersetzt sein können oder cyclisches Alkyl mit 3-7 C-Atomen,
- Hal: F, Cl, Br oder I,
- m: 1, 2, 3, 4, 5 oder 6,
- n: 0, 1 oder 2,
bedeuten,
sowie ihrer Säureadditionssalze,
dadurch gekennzeichnet, dass die Trennung an einem chiralen Ionenaustauschermaterial erfolgt.

Die Verbindungen der Formel I sind nützlich bei der Behandlung von mit dem Insulinresistenzsyndrom verbundenen Erkrankungen.

Bekannt ist ein Verfahren zur Racematspaltung von Verbindungen der Formel I aus der WO 2004/098817 durch Salzbildung und Trennung der diastereomeren Salze.

Überraschenderweise ergaben Untersuchungen im Rahmen der Trennung von Dihydro-1,3,5-triazinaminderivaten, dass die Verbindungen der Formel I im Vergleich zum Stand der Technik in erheblich höherer Ausbeute und in größerem Enantiomerenüberschuß erhalten werden können.

Nach dem erfindungsgemäßen Verfahren wird insbesondere die Verbindung 4-Amino-3,6-dihydro-2-dimethylamino-6-methyl-1,3,5-triazin hergestellt.

Im Gegensatz zum bisher bekannten Stand der Technik zur analytischen Trennung der Enantiomere mittels superkritischer HPLC an chiralen Phasen welcher in WO 2004/098817 beschrieben ist, konnte überraschenderweise doch ein analytisches Verfahren zur Bestimmung der beiden Enantiomere mittels Standard-HPLC gefunden werden. Dies hat den Vorteil, dass keine speziellen Geräte für die Trennung mehr nötig sind.

Die mobile Phase besteht typischerweise aus einem polaren Lösungsmittel wie z.B. Methanol, Ethanol, Wasser, Isopropanol und einem sauren oder basischen Puffersalz. Die mobile Phase enthält typischerweise 0,01% bis 2% dieses sauren oder basischen Puffersalzes.

Als stationäre Phase wird typischerweise ein chiraler Träger aus der Gruppe der Oligosaccharide, Polysaccharide oder auf Kieselgel gebundene makrozyklische Glycoproteine gewählt. Derartige Träger sind kommerziell erhältlich unter den Handelsnamen Chiralcel® von Daicel, Chirose® von Chiralsep sowie Chirobiotic® von Astec.

Vor- und nachstehend haben die Reste R¹, R², R³, R⁴, R⁵, R⁶ die bei der Formel I angegebenen Bedeutungen, sofern nicht ausdrücklich etwas anderes angegeben ist.

Formel I umfasst auch die optisch aktiven Formen (Stereoisomeren), wie die Enantiomeren.

### Metformin als bevorzugtes Edukt hat die Struktur

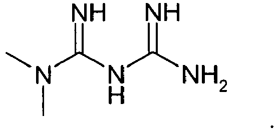

A bedeutet Alkyl, dieses ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet weiterhin bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

A bedeutet ganz besonders bevorzugt Methyl.

Cyclisches Alkyl (Cycloalkyl) bedeutet vorzugsweise Cyclopropyl, Cyclobutyl, Cylopentyl, Cyclohexyl oder Cycloheptyl.

Alkenyl hat 2, 3, 4, 5 oder 6 C-Atome und bedeutet vorzugsweise Vinyl, Propenyl oder Hexenyl.

Alkinyl hat 2, 3, 4, 5 oder 6 C-Atome und bedeutet vorzugsweise C≡CH oder C≡C-CH₃.

Ar bedeutet z.B. o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Aminophenyl, o-, m- oder p-(N-Methylamino)-phenyl, o-, m- oder p-(N-Methylamino-carbonyl)-phenyl, o-, m- oder p-Methoxyphenyl, o-, m- oder p-Ethoxyphenyl, o-, m- oder p-Ethoxycarbonylphenyl, o-, m- oder p-(N,N-Dimethyl-amino)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonyl)-phenyl, o-, m- oder p-Cyanphenyl, o-, m- oder p-Carboxyphenyl, o-, m- oder p-Methoxycarbonylphenyl, o-, m- oder p-Acetylphenyl weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Amino-4-chlor-, 2-Amino-3-chlor-, 2-Amino-4-chlor-, 2-Amino-5-chlor- oder 2-Amino-6-chlorphenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Fluor-4-methoxyphenyl, 3-Amino-6-methylphenyl oder 2,5-Dimethyl-4-chlorphenyl.

Ar bedeutet besonders bevorzugt Phenyl, Hydroxyphenyl oder Methoxyphenyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.

Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder-5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl, 2,3-Dihydro-2-oxo-furanyl, 3,4-Dihydro-2-oxo-1*H-*chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet vorzugsweise unsubstituiertes oder ein-, zwei- oder dreifach durch A, COOA, Hal und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl oder Benzo[2,1,3]thiadiazolyl.

R¹, R² bedeuten vorzugsweise A.

R³, R⁴ bedeuten vorzugsweise H.

R⁵ bedeutet vorzugsweise H.

R⁶ bedeutet vorzugsweise A.

Ganz besonders bevorzugt bedeuten
- R¹,: Methyl,
- R³, R⁴: H,
- R⁵: H,
- R⁶: Methyl.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Grundsätzlich lassen sich Enantiomere an chiralen Sorbentien trennen. Dem Fachmann sind eine große Anzahl chiraler Sorbentien, beispielsweise solche auf der Grundlage von Cellulosederivaten, Cyclodextrinen, oder Poly(meth)acrylamidderivaten mit optisch-aktiver Seitenkette bekannt. Solche chiralen Sorbentien und deren Verwendung sind beispielsweise in "Recent developments in liquid chromatographic enantioseparation. M. Lämmerhofer, W. Lindner in Handbook of analytical separations (Vol. 1): Separation methods in drug synthesis and purification (K. Valko, Editor), Elsevier, NL, 2000" oder in "Enantiomer Separation. M. Lämmerhofer, N. M. Maier, W. Lindner, in: L. R. Snyder, J. J. Kirkland, and J. W. Dolan (Editors), Introduction to Modern Liquid Chromatography, 3rd edition, John Wiley, Hoboken, NJ, USA, 2009" offenbart.

Überraschend wurde jedoch gefunden, dass eine Trennung dieser Verbindungsklasse auch an einem lonentauschermaterial möglich ist.

Derartige Austauschmaterialien zur enantioselektiven Trennung sind z.B. in WO 03/068397 A1 beschrieben.

Im Folgenden werden Charakteristika der enantioselektiven lonenaustauschermaterilien, die das erfindungsgemäße Problem lösen, beschrieben. Die verwendeten Ionenaustauschermaterialien sind im Wesentlichen aus einem i.) anionischem oder zwitterionischem chiralen Selektor, in) einem Träger, sowie iii) einem Linker, der den chiralen Selektor mit dem Träger verbindet, aufgebaut.

Der anionische oder zwitterionische chirale Selektor besteht aus einen chiralen Grundgerüst in enantiomeren-reiner Form mit zumindest einer Säurefunktion, wobei die Säurefunktion eine eine Carbon-, Sulfon-, Sulfin-, Phosphor-, Phosphon- oder Phosphinsäuregruppe ist. Diese Säuregruppe bewirkt die ionische Interaktion des chiralen Selektors bzw. Ionenaustauschmaterials mit der zu trennenden Verbindung der Formel I. Das chirale Grundgerüst enthält zumindest ein chirales Element aus der Gruppe der Chiralitätszentren, chiralen Achsen, chiralen Ebenen bzw. chiralen Helices und wird in Form eines definierten Stereoisomers eingesetzt, wobei die stereochemische Reinheit dieses chiralen Grundgerüsts möglichst hoch sein soll. Oft sind die chiralen Selektoren durch die Verknüpfung mehrerer Chiralitätszentren aufgebaut, was eine Anzahl wohl definierter Stereoisomerer bewirkt. Das chirale Element und die Kombination der chiralen Elemente des Grundgerüsts liefern die Basis für die chirale Erkennungsfähigkeit des enantioselektiven Ionenaustauschmaterials. Das chirale lonenaustauschgrundgerüst ist bevorzugt eine niedermolekulare Verbindung aus der Gruppe der natürlichen oder synthetischen, cyclischen oder nicht-cyclischen Aminosäuren, Hydroxycarbonsäuren, Aminosulfonsäuren, Aminophosphonsäuren, Aminophosphinsäuren, Aminosulfinsäuren, Hydroxyphosphonsäuren, Hydroxyphosphinsäuren, Ketosulfonsäuren, Weinsäure, Kamphersulfonsäure, Mandelsäure, sulfatierten Verbindungen, Peptiden oder Sulfopeptiden.

Der niedermolekular lonenaustausch-Selektor kann auch eine amphotäre Verbindung sein, welche bei den verwendeten Bedingungen zumindest eine geladene saure Gruppe trägt. Weitere typische Strukturelemente, welche einen erfolgreichen chiralen lonenaustausch-Selektor für die Enantiomerentrennung der Zielverbindungen der Formel I auszeichnen, sind zusätzliche Wasserstoff Donor-Akzeptor Gruppen (wie Amide, Carbamate, Sulfonamide, Harnstoff, Carbonyl, Semicarbazid, Hydrazid, or Sulfonimide Gruppen oder andere Wasserstoff Donor-Akzeptor Systeme), pi-pi-Interaktionsstellen (d.h. aromatische Gruppen vorzugsweise mit elektronen-anziehenden oder elektronen-abgebenden funktionellen Gruppen) sowie gegebenenfalls sperrige Gruppen für sterische Interaktionen oder van der Waals Wechselwirkung. Diese sekundären Wechselwirkungskräfte sind häufig stereoselektive ausgeprägt und bewirken die unterschiedliche Affinität der beiden Enantiomere der zu trennenden Zielverbindung.

Für die meisten Anwendungen muss der chirale Ionenaustausch Selektor an einem festen oder gegebenenfalls flüssigen Träger immobilisiert werden. Als Träger kommen anorganische, organische, oder gemischte anorganisch-organische Hybrid Materialien in Frage. Der Träger kann aus der Gruppe von partikulären oder monolithischen Materilien stammen, welche Kieselgel (SiO₂), Alumina (Al₂O₃), Zirconia (ZrO₂), Titania (TiO₂), andere Sol-Gel Materialien, organisch-anorganisch Kohlenstoff-Siliziumhältige Hybrid Materialien, optional quervernetzte Polysiloxane, optional quervernetzte organische Polymere aus der Gruppe der Poly(meth)acrylate, Poly(meth)acrylamide, Polystyrrole, "ring-opening methathesis" Polymere, Mischformen dieser organischen Polymere, Polysaccharide, Agarose und Keramik-Materialien beinhaltet. Die Träger sind bevorzugt poröse Materialien mit einer durchschnittlichen Porenweite von 60Å bis 1000Å, können aber auch nicht-porös oder superporös mit Porenweiten größer 1000Å sein.

Der Linker hat die Funktion den Selektor an der Oberfläche des Trägers zu verankern und den Selektor für die Interaktion mit der Zielverbindung zugänglich zu machen. Sowohl Länge als auch chemische Struktur es Linkers sind variabel, da dieser in der Regel nur indirekt an der chiralen Erkennung und der Trennung beteiligt ist. Gegebenenfalls ist kein Linker notwendig, nämlich dann, wenn der lonenaustausch-Selektor in monomerer oder polymerer Form direkt auf die Oberfläche des Trägers gecoated ist. All herkömmlichen Festphasen-Linker Konzepte können verwendet werden. Typische Immobilisierungsstrategien verwenden einen bifunktionellen Linker der zuerst mit einer funktionellen Gruppe am Träger verankert wird und im zweiten Schritt via reaktiver Ankergruppe des modifizierten Trägers mit dem chiralen lonenaustausch-Selektors zur chemischen Reaktion gebracht und somit am Träger immobilisiert wird. Ionenaustausch-Selektoren mit einer Doppelbindung können so z. B. an Thiol-modifiziertes Kieselgel kovalent gebunden werden.

Neben der nachträglichen bürstenartigen Verankerung an der Oberfläche des Trägers sind auch Konzepte wie Graft-Polymerisation und ähnliche polymere Verankerungsstrategien vorstellbar. Der chirale lonenaustausch-Selektor kann auch durch *in-situ* Co-Polymerisation in den Träger eingebettet und so verankert werden.

Die resultierenden Belegungsdichten liegen bevorzugt zwischen 100 und 1000 µmol lonenaustausch-Selektor/g stationärer Phase.

### Als Elutionsmittel wird vorzugsweise

i) ein organisches Lösungsmittel aus der Gruppe Methanol, Ethanol, Propanol, Acetonitril, THF, Dioxan, Ethylacetat, Chloroform, Dichlormethan, tert-Butylmethylether, Hexan, Heptan, oder eine binäre, ternäre, quaternäre Mischung dieser Lösungsmittel unter Zusatz von Co- und Gegen-Ionen oder auch ohne ionogene Zusätze,
ii) ein wässriges Medium mit oder ohne Pufferzusätze sowie mit oder ohne mischbare polare organische Lösungsmittel aus der Gruppe wie unter i.) spezifiziert,
iii) superkritisches bzw. subkritisches CO₂ mit oder ohne einem organischen Lösungsmittel wie unter i.) spezifiziert, unter Zusatz von Co- und Gegen-Ionen oder auch ohne ionogene Zusätze
verwendet.

Die verwendeten Additive sind bevorzugt flüchtig. Die Elution der Komponenten wird bevorzugt im isokratischen Modus bewerkstelligt, kann aber auch im Gradientenelutionsmodus erfolgen.

Die Trennung kann in einem herkömmlichen Zonenelutionschromatographie-Verfahren mit diskontinuierlicher Probenaufgabe und kontinuierlicher Elution, im Batch-Modus, durch Recycling- Chromatographie, oder durch ein kontinuierliches Chromatographieverfahren (wie Simulated Moving Bed SMB Technologie) erfolgen. Die chromatographische Trennung kann mittels HPLC, UPLC, SFC Technologie bewerkstelligt werden.

Das erfindungsgemäße Verfahren liefert Ausbeuten von fast 50% mit einem ee > 98% bezogen auf das eingesetzte zu trennende Racemische Gemisch.

### Beispiel 1)

Für das erfindungsgemäße Verfahren kann beispielsweise das chirale lonentauschermaterial mit der folgenden chemischen Struktur, basierend auf Kieselgel als Trägermaterial, eingesetzt werden. Die Art des Gegenions X⁻ zur Kationentauscherstelle hängt ab von der Art des Puffersalzes in der mobilen Phase.

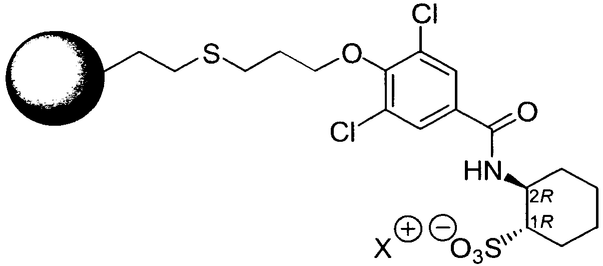

Unter Verwendung dieses lonentauschermaterials kann folgende chromatographische Trennung des Racemats der Verbindung der Formel I, worin
- R¹, R²: Methyl,
- R³, R⁴: H,
- R⁵: H,
- R⁶: Methyl
bedeuten,
erzielt werden.

Experimentelle Bedingungen: Säulendimension 150 x 4 mm I.D., Partikelgröße 5 µm, Temperatur 25°C, Fließgeschwindigkeit 1.0 ml/min, Detektion 240 nm, mobilen Phase 50 mM Ameisensäure und 25 mM Diethylamin in Acetonitril/Methanol 9/1 (v/v).

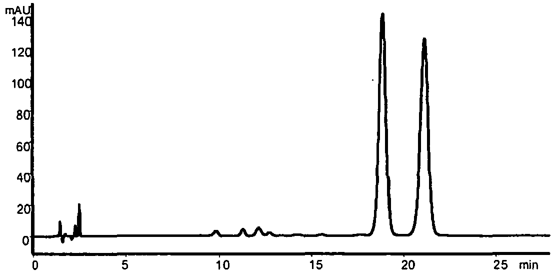

Die Elutionsreihenfolge kann durch Wechsel zum Ionentauschermaterial mit enantiomerer Absolutkonfiguration umgekehrt werden. 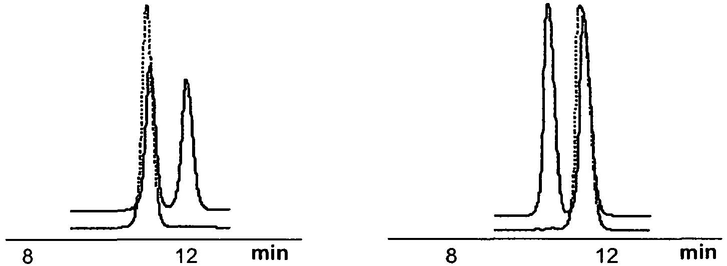
links: oben gezeigtes lonentauschermaterial mit (1 R,2R)-Konfiguration
rechts: oben gezeigtes lonentauschermaterial mit (1 S,2S)-Konfiguration
durchgezogene Linie: Trennung des Racemats, gestrichelte Linie: Elution eines einzelnen Enantiomers
Experimentelle Bedingungen: Säulendimension 100 x 4 mm I.D., Partikelgröße 5 µm, Temperatur 25°C, Fließgeschwindigkeit 1.0 ml/min, Detektion 240 nm, mobile Phase: 10 mM NH4Cl in Methanol

### Beispiel 2)

Weitere Beispiele für das erfindungsgemäße Verfahren mittels des oben genannten lonentauschermaterials zur Trennung des Racemats der Verbindung der Formel I, worin in a)
- R¹: Allyl,
- R²: Methyl,
- R³, R⁴: H,
- R⁵: H,
- R⁶: Methyl
und in b)
- R¹, R²: Methyl,
- R³,: Allyl,
- R⁴: H,
- R⁵: H,
- R⁶: Methyl
bedeuten:

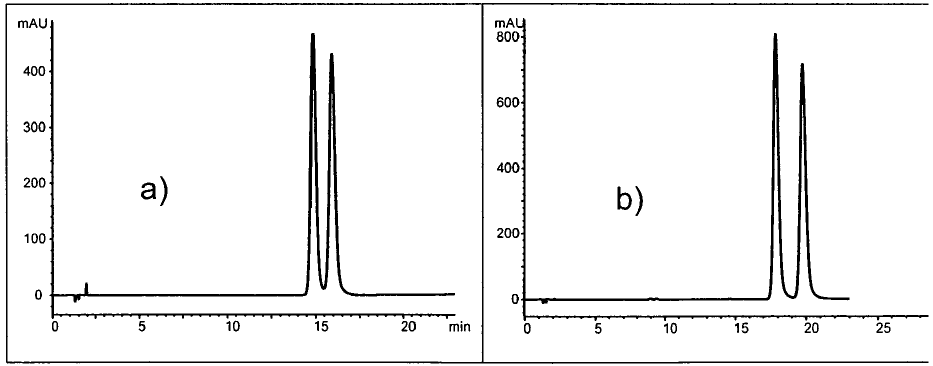

Experimentelle Bedingungen: Säulendimension 150 x 4 mm I.D., Partikelgröße 5 µm, Temperatur 25°C, Fließgeschwindigkeit 1.0 ml/min, Detektion 240 nm, mobile Phase: 50 mM Ameisensäure und 25 mM Diethylamin in Methanol.

### Beispiel 3)

Weitere Beispiele für das erfindungsgemäße Verfahren mittels zwitterionischen lonentauschermaterials mit der chemischen Struktur 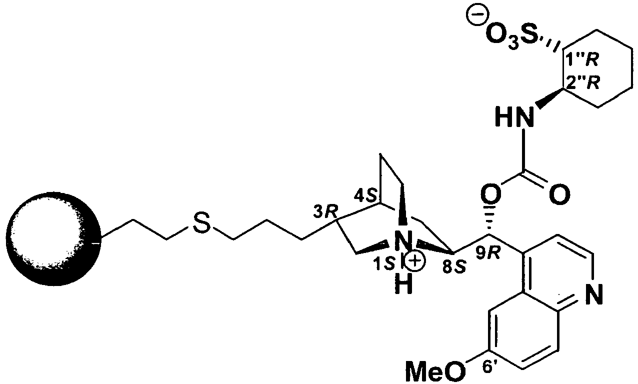 zur Trennung des Racemats der Verbindung der Formel I, worin in a)
- R¹, R²: Methyl,
- R³, R⁴: H,
- R⁵: H,
- R⁶: Methyl
und in b)
- R¹, R²: Methyl,
- R³,: Allyl,
- R⁴: H,
- R⁵: H,
- R⁶: Methyl
bedeuten:

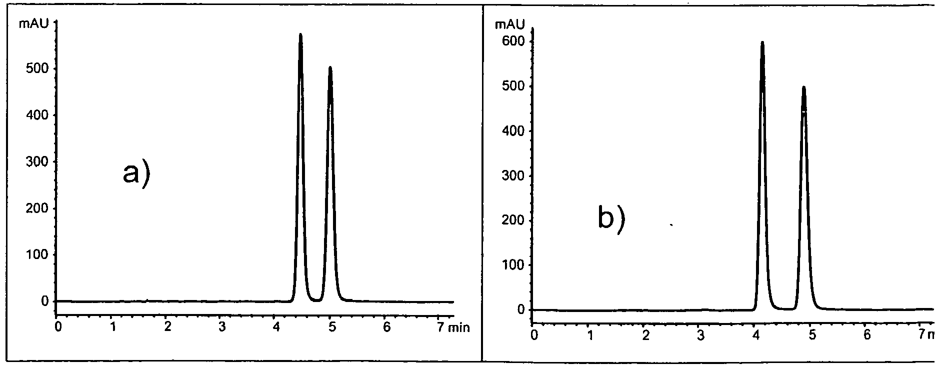

Experimentelle Bedingungen: Säulendimension 150 x 4 mm I.D., Partikelgröße 5 µm, Temperatur 25°C, Fließgeschwindigkeit 1.0 ml/min, Detektion 240 nm, mobile Phase: 50 mM Essigsäure und 25 mM Ammoniak in Methanol.

### Beispiel 4)

Beispiel für das erfindungsgemäße Verfahren für die präparative Trennung des Racemats der Verbindung der Formel I, worin
- R¹: Allyl,
- R²: Methyl,
- R³, R⁴: H,
- R⁵: H,
- R⁶: Methyl
bedeuten,
mit dem chiralen lonentauschermaterial der folgenden chemischen Struktur, basierend auf Kieselgel als Trägermaterial; die Art des Gegenions X⁻ zur Kationentauscherstelle hängt ab von der Art des Puffersalzes in der mobilen Phase.

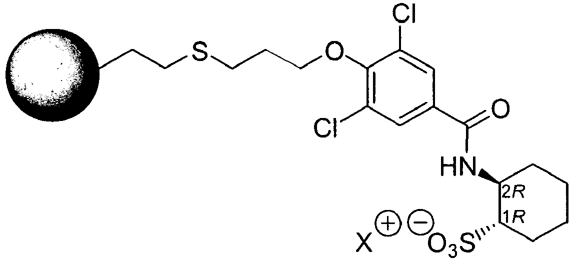

### Experimentelle Bedingungen für das folgende Chromatogramm:

Säulendimension 150 x 4 mm I.D., Partikelgröße 5 µm, Temperatur 25°C, Fließgeschwindigkeit 1.0 ml/min, Detektion 254 nm (durchgezogene Linie), 280 nm (gestrichelte Linie), mobile Phase: 50 mM Essigsäure und 25 mM Ammoniak in Acetonitril/Methanol 4/1 (v/v), Probenkonzentration 113 mg/ml, Injektionsvolumen 26,5 µl.

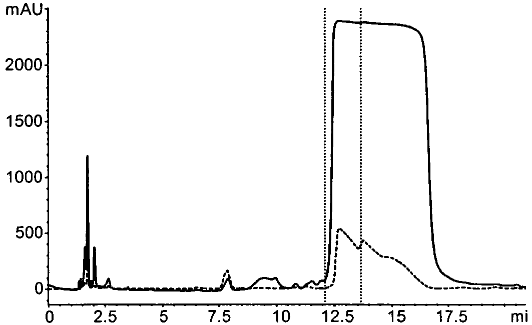

Die gesammelte Fraktion (12,20 - 13,85 min) für das zuerst eluierende Enantiomer ist durch die gepunkteten senkrechten Linien zeitlich angezeigt.

Durch Analyse auf dem chiralen lonentauschermaterial kann die Enantiomerenreinheit der gesammelten Fraktion bestimmt werden.
Experimentelle Bedingungen für das folgende Chromatogramm: Absolutkonfiguration des lonentauschermaterials (1 S,2S), Säulendimension 150 x 4 mm I.D., Partikelgröße 5 µm, Temperatur 25°C, Fließgeschwindigkeit 1.0 ml/min, Detektion 254 nm, mobile Phase: 50 mM Essigsäure und 25 mM Ammoniak in Methanol.
durchgezogene Linie: Analyse der gesammten Fraktion auf >98% ee bei einer Ausbeute von >80% bezogen auf das Enantiomergestrichelte Linie: Trennung des entsprechenden Racemats zum Vergleich

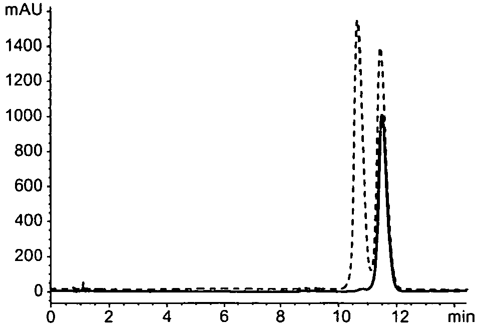

### Beispiel 5)

Beispiele für chirale Verbindungen, die die Trennung von Racematen der Verbindungen der Formel I erlauben, beispielsweise in gelöster Form als Zusatz zum Hintergrundelektrolyt in der Kapillarelektrophorese können folgende Strukturen aufweisen:

| chemische Struktur | Enantioselektivität | chemische Struktur | Enantioselektivität |
|---|---|---|---|
| 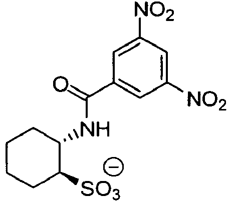 | **1.075** | 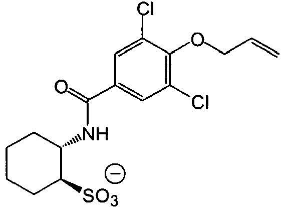 | **1.030** |
| 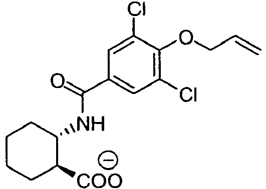 | **1.015** | 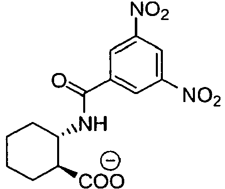 | **1.028** |
| 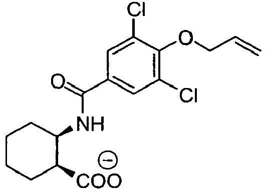 | **1.006** | 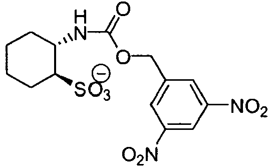 | **1.012** |
| 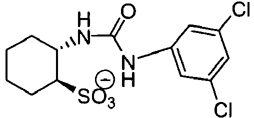 | **1.033** | 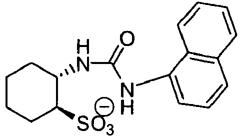 | **1.024** |
| 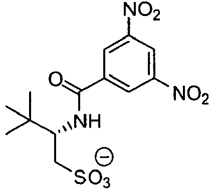 | **1.046** | 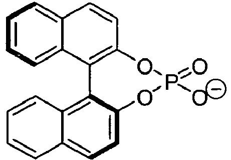 | **1.037** |

### Experimentelle Bedingungen des reziproken CE Experiments:

Hintergrundelektrolyt: 50 mM Ameisensäure + 25 mM Triethylamin + 50 mM 4-Amino-3,6-dihydro-2-dimethylamino-6-methyl-1,3,5-triazin (enantiomeren-rein) in Ethanol; T = 25°C; Injektion: 50 mbar/5s; Proben: Verbindungen der Tabelle in razemischer Form (1-10 mg/mL in Elektrolyt); Fused-Silica Kapillare: 50 µm Innendurchmesser; Gesamtlänge = 50 cm; effektive Länge bis zum Detektor = 41,5 cm; Spannung = -25 kV; nach 30 Minuten wird auf der Injektorseite ein Druck von 20 mbar angelegt, um nicht eluierte Verbindungen aus der Kapillare zu entfernen.

Nach geeigneter Verankerung dieser Verbindungen an einem Träger, wie partikuläres oder monolithisches Kieselgel, oder partikuläres oder monolithische organische Polymere, können Ionenaustauscher erhalten werden, welche die Trennung der chiralen Verbindungen der Formel I in ihre Enantiomere ermöglichen.

## Patentansprüche

1. Verfahren zur chromatographischen Trennung von Verbindungen der Formel I 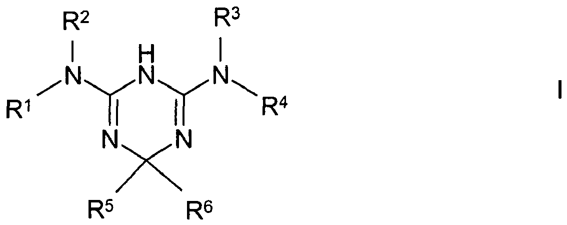 worin
R¹, R² jeweils unabhängig voneinander H oder A,
R³, R⁴ jeweils unabhängig voneinander H, A, Alkenyl mit 2-10 C-Atomen, Alkinyl mit 2-10 C-Atomen, Ar oder Het,
R⁵ und R⁶ zusammen auch Alkylen mit 2, 3, 4 oder 5 C-Atomen,
R⁵, R⁶ jeweils unabhängig voneinander H, A, (CH₂)ₙAr, (CH₂)ₘOAr, (CH₂)ₘOA oder (CH₂)ₘOH,
R⁵ und R⁶ zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
Ar unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
A unverzweigtes oder verzweigtes Alkyl mit 1-10 C-Atomen, worin 1-7 H-Atome durch F ersetzt sein können oder cyclisches Alkyl mit 3-7 C-Atomen,
Hal F, Cl, Br oder I,
m 1, 2, 3, 4, 5 oder 6,
n 0, 1 oder 2,
bedeuten,
sowie ihrer Säureadditionssalze,
**dadurch gekennzeichnet, dass** die Trennung an einem chiralen Ionenaustauscher erfolgt.

2. Verfahren nach Anspruch 1, **dadurch gekennzeichnet, dass** die Trennung der Enantiomere der Formel I,
worin
R¹, R² jeweils unabhängig voneinander H oder A,
R³, R⁴ jeweils unabhängig voneinander H, A, Alkenyl mit 2-10 C-Atomen, Alkinyl mit 2-10 C-Atomen, Ar oder Het,
R⁵ und R⁶ zusammen auch Alkylen mit 2, 3, 4 oder 5 C-Atomen,
R⁵, R⁶ jeweils unabhängig voneinander H, A, (CH₂)ₙAr, (CH₂)ₘOAr, (CH₂)ₘOA oder (CH₂)ₘOH,
R⁵ und R⁶ zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
Ar unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
A unverzweigtes oder verzweigtes Alkyl mit 1-10 C-Atomen, worin 1-7 H-Atome durch F ersetzt sein können oder
cyclisches Alkyl mit 3-7 C-Atomen,
Hal F, Cl, Br oder I,
m 1, 2, 3, 4, 5 oder 6,
n 0, 1 oder 2,
an einem Anionischen Austauschermaterial im Zuge eines Kationenaustauschs erfolgt.

3. Verfahren nach Anspruch 1 oder 2, **dadurch gekennzeichnet, dass** Enantiomere der Formel I,
worin
R¹, R² jeweils unabhängig voneinander H oder A,
R³, R⁴ jeweils unabhängig voneinander H, A, Alkenyl mit 2-10 C-Atomen, Alkinyl mit 2-10 C-Atomen, Ar oder Het,
R⁵ und R⁶ zusammen auch Alkylen mit 2, 3, 4 oder 5 C-Atomen,
R⁵, R⁶ jeweils unabhängig voneinander H, A, (CH₂)ₙAr, (CH₂)ₘOAr, (CH₂)ₘOA oder (CH₂)ₘOH,
R⁵ und R⁶ zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
Ar unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
A unverzweigtes oder verzweigtes Alkyl mit 1-10 C-Atomen, worin 1-7 H-Atome durch F ersetzt sein können oder
cyclisches Alkyl mit 3-7 C-Atomen,
Hal F, Cl, Br oder I,
m 1, 2, 3, 4, 5 oder 6,
n 0, 1 oder 2,
bedeuten,
an zwitterionischen chiralen Stationären Phasen getrennt werden.

4. Verfahren nach Anspruch 1 bis 3, **dadurch gekennzeichnet, dass** Enantiomere der Formel I,
worin
R¹, R² jeweils unabhängig voneinander H oder A,
R³, R⁴ jeweils unabhängig voneinander H, A, Alkenyl mit 2-10 C-Atomen, Alkinyl mit 2-10 C-Atomen, Ar oder Het,
R⁵ und R⁶ zusammen auch Alkylen mit 2, 3, 4 oder 5 C-Atomen,
R⁵, R⁶ jeweils unabhängig voneinander H, A, (CH₂)ₙAr, (CH₂)ₘOAr, (CH₂)ₘOA oder (CH₂)ₘOH,
R⁵ und R⁶ zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
Ar unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
A unverzweigtes oder verzweigtes Alkyl mit 1-10 C-Atomen, worin 1-7 H-Atome durch F ersetzt sein können oder
cyclisches Alkyl mit 3-7 C-Atomen,
Hal F, Cl, Br oder I,
m 1, 2, 3, 4, 5 oder 6,
n 0, 1 oder 2,
bedeuten,
an Stationären Phasen mit Ionischen Wechselwirkungen unterstützt durch Wasserstoffbrückenbindungen getrennt werden.

5. Verfahren nach Anspruch 1 bis 4, **dadurch gekennzeichnet, dass** Enantiomere der Formel I,
worin
R¹ , R² A
bedeuten,
getrennt werden.

6. Verfahren nach Anspruch 1 bis 5, **dadurch gekennzeichnet, dass** Enantiomere der Formel I,
worin
R³, R⁴ H
bedeuten,
getrennt werden.

7. Verfahren nach Anspruch 1 bis 6, **dadurch gekennzeichnet, dass** Enantiomere der Formel I,
worin
R⁵ H,
R⁶ A
bedeuten,
getrennt werden.

8. Verfahren nach Anspruch 1 bis 7, **dadurch gekennzeichnet, dass** Enantiomere der Formel I,
worin
R¹, R² Methyl,
R³, R⁴ H,
R⁵ H,
R⁶ Methyl
bedeuten,
getrennt werden.

9. Verfahren nach Anspruch 1 bis 8, **dadurch gekennzeichnet, dass** Enantiomere der Formel I,
worin
R¹, R² Methyl,
R³, R⁴ H,
R⁵ H,
R⁶ Methyl
bedeuten,
getrennt werden.

10. Verfahren nach Anspruch 1 bis 9, **dadurch gekennzeichnet, dass** das Ionenaustauschermaterial einen chiralen Selektor, der aus einer chiralen Komponente und zumindest einer Ionenaustauschgruppe zusammengesetzt ist, einen Spacer und einen Träger umfasst.

11. Verfahren nach Anspruch 1 bis 10, **dadurch gekennzeichnet, dass** die chirale Komponete ein Molekulargewicht von weniger als 1.000 hat und die Kationenaustauschgruppe eine Säuregruppe mit einem pKa<4,0 ist.

12. Verfahren nach Anspruch 11, **dadurch gekennzeichnet, dass** die Säuregruppe eine Carbon-, Sulfon-, Sulfin-, Phosphor-, Phosphon- oder Phosphinsäuregruppe ist.

13. Verfahren nach Anspruch 1-12, **dadurch gekennzeichnet, dass** ein Elutionsmittel, enthaltend
i) ein organisches Lösungsmittel aus der Gruppe Methanol, Ethanol, Propanol, Acetonitril, THF, Dioxan, Ethylacetat, Chloroform, Dichlormethan, tert-Butylmethylether, Hexan, Heptan, oder eine binäre, ternäre, quaternäre Mischung dieser Lösungsmittel unter Zusatz von Co- und Gegen-Ionen oder auch ohne ionogene Zusätze,
ii) ein wässriges Medium mit oder ohne Pufferzusätze sowie mit oder ohne mischbare polare organische Lösungsmittel aus der Gruppe wie unter i.) spezifiziert,
iii) superkritisches bzw. subkritisches CO₂ mit oder ohne einem organischen Lösungsmittel wie unter i.) spezifiziert, unter Zusatz von Co- und Gegen-Ionen oder auch ohne ionogene Zusätze verwendet wird.

## Claims

1. Method for the chromatographic separation of compounds of the formula I 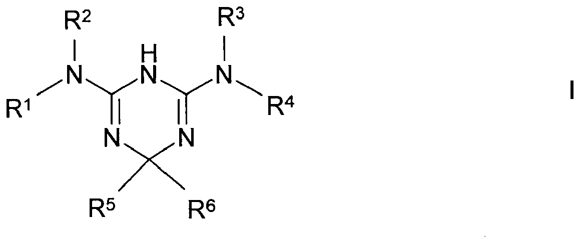 in which
R¹, R² each, independently of one another, denote H or A,
R³, R⁴ each, independently of one another, denote H, A, alkenyl having 2-10 C atoms, alkynyl having 2-10 C atoms, Ar or Het,
R⁵ and R⁶ together also denote alkylene having 2, 3, 4 or 5 C atoms,
R⁵, R⁶ each, independently of one another, denote H, A, (CH₂)ₙAr, (CH₂)ₘOAr, (CH₂)ₘOA or (CH₂)ₘOH,
R⁵ and R⁶ together also denote alkylene having 2, 3, 4 or 5 C atoms,
in which one CH₂ group may be replaced by O, NH or NA and/or in which 1 H atom may be replaced by OH,
Ar denotes phenyl, naphthyl or biphenyl, each of which is unsubstituted or mono-, di- or trisubstituted by Hal, A, OA, OH, COOH, COOA, CN, NH₂, NHA, NA₂, SO₂A and/or COA,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OH, OA, NH₂, (CH₂)ₙAr, NHA, NA₂, COOH, COOA and/or =0 (carbonyl oxygen),
A denotes unbranched or branched alkyl having 1-10 C atoms, in which 1-7 H atoms may be replaced by F, or cyclic alkyl having 3-7 C atoms,
Hal denotes F, Cl, Br or I,
m denotes 1, 2, 3, 4, 5 or 6,
n denotes 0, 1 or 2,
and acid-addition salts thereof,
**characterised in that** the separation is carried out on a chiral ion exchanger.

2. Method according to Claim 1, **characterised in that** the separation of the enantiomers of the formula I
in which
R¹, R² each, independently of one another, denote H or A,
R³, R⁴ each, independently of one another, denote H, A, alkenyl having 2-10 C atoms, alkynyl having 2-10 C atoms, Ar or Het,
R⁵ and R⁶ together also denote alkylene having 2, 3, 4 or 5 C atoms,
R⁵, R⁶ each, independently of one another, denote H, A, (CH₂)ₙAr, (CH₂)ₘOAr, (CH₂)ₘOA or (CH₂)ₘOH,
R⁵ and R⁶ together also denote alkylene having 2, 3, 4 or 5 C atoms,
in which one CH₂ group may be replaced by O, NH or NA and/or in which 1 H atom may be replaced by OH,
Ar denotes phenyl, naphthyl or biphenyl, each of which is unsubstituted or mono-, di- or trisubstituted by Hal, A, OA, OH, COOH, COOA, CN, NH₂, NHA, NA₂, SO₂A and/or COA,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OH, OA, NH₂, (CH₂)ₙAr, NHA, NA₂, COOH, COOA and/or =O (carbonyl oxygen),
A denotes unbranched or branched alkyl having 1-10 C atoms, in which 1-7 H atoms may be replaced by F, or cyclic alkyl having 3-7 C atoms,
Hal denotes F, Cl, Br or I,
m denotes 1, 2, 3, 4, 5 or 6,
n denotes 0, 1 or 2,
is carried out on an anionic exchanger material in the course of a cation exchange.

3. Method according to Claim 1 or 2, **characterised in that** enantiomers of the formula I
in which
R¹, R² each, independently of one another, denote H or A,
R³, R⁴ each, independently of one another, denote H, A, alkenyl having 2-10 C atoms, alkynyl having 2-10 C atoms, Ar or Het,
R⁵ and R⁶ together also denote alkylene having 2, 3, 4 or 5 C atoms,
R⁵, R⁶ each, independently of one another, denote H, A, (CH₂)ₙAr, (CH₂)ₘOAr, (CH₂)ₘOA or (CH₂)ₘOH,
R⁵ and R⁶ together also denote alkylene having 2, 3, 4 or 5 C atoms,
in which one CH₂ group may be replaced by O, NH or NA and/or in which 1 H atom may be replaced by OH,
Ar denotes phenyl, naphthyl or biphenyl, each of which is unsubstituted or mono-, di- or trisubstituted by Hal, A, OA, OH, COOH, COOA, CN, NH₂, NHA, NA₂, SO₂A and/or COA,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OH, OA, NH₂, (CH₂)ₙAr, NHA, NA₂, COOH, COOA and/or =O (carbonyl oxygen),
A denotes unbranched or branched alkyl having 1-10 C atoms, in which 1-7 H atoms may be replaced by F, or cyclic alkyl having 3-7 C atoms,
Hal denotes F, Cl, Br or I,
m denotes 1, 2, 3, 4, 5 or 6,
n denotes 0, 1 or 2,
are separated on zwitterionic chiral stationary phases.

4. Method according to Claims 1 to 3, **characterised in that** enantiomers of the formula I
in which
R¹, R² each, independently of one another, denote H or A,
R³, R⁴ each, independently of one another, denote H, A, alkenyl having 2-10 C atoms, alkynyl having 2-10 C atoms, Ar or Het,
R⁵ and R⁶ together also denote alkylene having 2, 3, 4 or 5 C atoms,
R⁵, R⁶ each, independently of one another, denote H, A, (CH₂)ₙAr, (CH₂)ₘOAr, (CH₂)ₘOA or (CH₂)ₘOH,
R⁵ and R⁶ together also denote alkylene having 2, 3, 4 or 5 C atoms,
in which one CH₂ group may be replaced by O, NH or NA and/or in which 1 H atom may be replaced by OH,
Ar denotes phenyl, naphthyl or biphenyl, each of which is unsubstituted or mono-, di- or trisubstituted by Hal, A, OA, OH, COOH, COOA, CN, NH₂, NHA, NA₂, SO₂A and/or COA,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OH, OA, NH₂, (CH₂)ₙAr, NHA, NA₂, COOH, COOA and/or =O (carbonyl oxygen),
A denotes unbranched or branched alkyl having 1-10 C atoms, in which 1-7 H atoms may be replaced by F, or cyclic alkyl having 3-7 C atoms,
Hal denotes F, Cl, Br or I,
m denotes 1, 2, 3, 4, 5 or 6,
n denotes 0, 1 or 2,
are separated on stationary phases with ionic interactions supported by hydrogen bonds.

5. Method according to Claims 1 to 4, **characterised in that** enantiomers of the formula I
in which
R¹, R² denote A,
are separated.

6. Method according to Claims 1 to 5, **characterised in that** enantiomers of the formula I
in which
R³, R⁴ denote H,
are separated.

7. Method according to Claims 1 to 6, **characterised in that** enantiomers of the formula I
in which
R⁵ denotes H,
R⁶ denotes A,
are separated.

8. Method according to Claims 1 to 7, **characterised in that** enantiomers of the formula I
in which
R¹, R² denote methyl,
R³, R⁴ denote H,
R⁵ denotes H,
R⁶ denotes methyl,
are separated.

9. Method according to Claims 1 to 8, **characterised in that** enantiomers of the formula I
in which
R¹, R² denote methyl,
R³, R⁴ denote H,
R⁵ denotes H,
R⁶ denotes methyl,
are separated.

10. Method according to Claims 1 to 9, **characterised in that** the ion-exchanger material comprises a chiral selector, which is composed of a chiral component and at least one ion-exchange group, a spacer and a support.

11. Method according to Claims 1 to 10, **characterised in that** the chiral component has a molecular weight of less than 1000, and the cation-exchange group is an acid group having a pKa < 4.0.

12. Method according to Claim 11, **characterised in that** the acid group is a carboxylic, sulfonic, sulfinic, phosphoric, phosphonic or phosphinic acid group.

13. Method according to Claims 1-12, **characterised in that** an eluent comprising
i) an organic solvent from the group methanol, ethanol, propanol, acetonitrile, THF, dioxane, ethyl acetate, chloroform, dichloromethane, tert-butyl methyl ether, hexane, heptane, or a binary, ternary, quaternary mixture of these solvents with addition of co- and counterions or also without ionogenic additives,
ii) an aqueous medium with or without addition of buffers and with or without miscible polar organic solvents from the group as specified under i.),
iii) supercritical or subcritical CO₂ with or without an organic solvent as specified under i.), with addition of co- and counterions or also without ionogenic additives,
is used.

## Revendications

1. Méthode de séparation chromatographique de composés de formule I 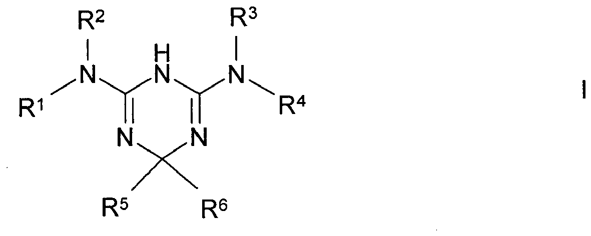 dans laquelle
R¹, R² désignent chacun, indépendamment l'un de l'autre, H ou A,
R³, R⁴ désignent chacun, indépendamment l'un de l'autre, H, A, alcényle ayant 2-10 atomes de C, alcynyle ayant 2-10 atomes de C, Ar ou Hét,
R⁵ et R⁶ désignent aussi ensemble alkylène ayant 2, 3, 4 ou 5 atomes de C,
R⁵, R⁶ désignent chacun, indépendamment l'un de l'autre, H, A, (CH₂)ₙAr, (CH₂)ₘOAr, (CH₂)ₘOA ou (CH₂)ₘOH,
R⁵ et R⁶ désignent aussi ensemble alkylène ayant 2, 3, 4 ou 5 atomes de C,
où un groupement CH₂ peut être remplacé par O, NH ou NA et/ou où 1 atome de H peut être remplacé par OH,
Ar désigne phényle, naphtyle ou biphényle, chacun d'entre eux étant non substitué ou mono-, di- ou trisubstitué par Hal, A, OA, OH, COOH, COOA, CN, NH₂, NHA, NA₂, SO₂A et/ou COA,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OH, OA, NH₂, (CH₂)ₙAr, NHA, NA₂, COOH, COOA et/ou =O (oxygène du carbonyle),
A désigne alkyle non ramifié ou ramifié ayant 1-10 atomes de C, où 1-7 atomes de H peuvent être remplacés par F, ou
cyclique alkyle ayant 3-7 atomes de C,
Hal désigne F, CI, Br ou I,
m désigne 1, 2, 3, 4, 5 ou 6,
n désigne 0, 1 ou 2,
et des sels d'addition d'acide de ceux-ci,
**caractérisée en ce que** la séparation est effectuée sur un échangeur d'ions chiral.

2. Méthode selon la revendication 1, **caractérisée en ce que** la séparation des énantiomères de formule I
dans laquelle
R¹, R² désignent chacun, indépendamment l'un de l'autre, H ou A,
R³, R⁴ désignent chacun, indépendamment l'un de l'autre, H, A, alcényle ayant 2-10 atomes de C, alcynyle ayant 2-10 atomes de C, Ar ou Hét,
R⁵ et R⁶ désignent aussi ensemble alkylène ayant 2, 3, 4 ou 5 atomes de C,
R⁵, R⁶ désignent chacun, indépendamment l'un de l'autre, H, A, (CH₂)ₙAr, (CH₂)ₘOAr, (CH₂)ₘOA ou (CH₂)ₘOH,
R⁵ et R⁶ désignent aussi ensemble alkylène ayant 2, 3, 4 ou 5 atomes de C,
où un groupement CH₂ peut être remplacé par O, NH ou NA et/ou où 1 atome de H peut être remplacé par OH,
Ar désigne phényle, naphtyle ou biphényle, chacun d'entre eux étant non substitué ou mono-, di- ou trisubstitué par Hal, A, OA, OH, COOH, COOA, CN, NH₂, NHA, NA₂, SO₂A et/ou COA,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OH, OA, NH₂, (CH₂)ₙAr, NHA, NA₂, COOH, COOA et/ou =O (oxygène du carbonyle),
A désigne alkyle non ramifié ou ramifié ayant 1-10 atomes de C, où 1-7 atomes de H peuvent être remplacés par F, ou
cyclique alkyle ayant 3-7 atomes de C,
Hal désigne F, CI, Br ou I,
m désigne 1, 2, 3, 4, 5 ou 6,
n désigne 0, 1 ou 2,
est effectuée sur un matériau d'échange anionique au cours d'un échange de cations.

3. Méthode selon la revendication 1 ou 2, **caractérisée en ce que** les énantiomères de formule I
dans laquelle
R¹, R² désignent chacun, indépendamment l'un de l'autre, H ou A,
R³, R⁴ désignent chacun, indépendamment l'un de l'autre, H, A, alcényle ayant 2-10 atomes de C, alcynyle ayant 2-10 atomes de C, Ar ou Hét,
R⁵ et R⁶ désignent aussi ensemble alkylène ayant 2, 3, 4 ou 5 atomes de C,
R⁵, R⁶ désignent chacun, indépendamment l'un de l'autre, H, A, (CH₂)ₙAr, (CH₂)ₘOAr, (CH₂)ₘOA ou (CH₂)ₘOH,
R⁵ et R⁶ désignent aussi ensemble alkylène ayant 2, 3, 4 ou 5 atomes de C, où un groupement CH₂ peut être remplacé par O, NH ou NA et/ou où 1 atome de H peut être remplacé par OH,
Ar désigne phényle, naphtyle ou biphényle, chacun d'entre eux étant non substitué ou mono-, di- ou trisubstitué par Hal, A, OA, OH, COOH, COOA, CN, NH₂, NHA, NA₂, SO₂A et/ou COA,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OH, OA, NH₂, (CH₂)ₙAr, NHA, NA₂, COOH, COOA et/ou =0 (oxygène du carbonyle),
A désigne alkyle non ramifié ou ramifié ayant 1-10 atomes de C, où 1-7 atomes de H peuvent être remplacés par F, ou cyclique alkyle ayant 3-7 atomes de C,
Hal désigne F, Cl, Br ou I,
m désigne 1, 2, 3, 4, 5 ou 6,
n désigne 0, 1 ou 2,
sont séparés sur des phases stationnaires chirales zwitterioniques.

4. Méthode selon les revendications 1 à 3, **caractérisée en ce que** les énantiomères de formule I
dans laquelle
R¹, R² désignent chacun, indépendamment l'un de l'autre, H ou A,
R³, R⁴ désignent chacun, indépendamment l'un de l'autre, H, A, alcényle ayant 2-10 atomes de C, alcynyle ayant 2-10 atomes de C, Ar ou Hét,
R⁵ et R⁶ désignent aussi ensemble alkylène ayant 2, 3, 4 ou 5 atomes de C,
R⁵, R⁶ désignent chacun, indépendamment l'un de l'autre, H, A, (CH₂)ₙAr, (CH₂)ₘOAr, (CH₂)ₘOA ou (CH₂)ₘOH,
R⁵ et R⁶ désignent aussi ensemble alkylène ayant 2, 3, 4 ou 5 atomes de C,
où un groupement CH₂ peut être remplacé par O, NH ou NA et/ou où 1 atome de H peut être remplacé par OH,
Ar désigne phényle, naphtyle ou biphényle, chacun d'entre eux étant non substitué ou mono-, di- ou trisubstitué par Hal, A, OA, OH, COOH, COOA, CN, NH₂, NHA, NA₂, SO₂A et/ou COA,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OH, OA, NH₂, (CH₂)ₙAr, NHA, NA₂, COOH, COOA et/ou =O (oxygène du carbonyle),
A désigne alkyle non ramifié ou ramifié ayant 1-10 atomes de C, où 1-7 atomes de H peuvent être remplacés par F, ou
cyclique alkyle ayant 3-7 atomes de C,
Hal désigne F, CI, Br ou I,
m désigne 1, 2, 3, 4, 5 ou 6,
n désigne 0, 1 ou 2,
sont séparés sur des phases stationnaires ayant des interactions ioniques supportées par des liaisons hydrogène.

5. Méthode selon les revendications 1 à 4, **caractérisée en ce que** les énantiomères de formule I
dans laquelle
R¹, R² désignent A,
sont séparés.

6. Méthode selon les revendications 1 à 5, **caractérisée en ce que** les énantiomères de formule I
dans laquelle
R³, R⁴ désignent H,
sont séparés.

7. Méthode selon les revendications 1 à 6, **caractérisée en ce que** les énantiomères de formule I
dans laquelle
R⁵ désigne H,
R⁶ désigne A,
sont séparés.

8. Méthode selon les revendications 1 à 7, **caractérisée en ce que** les énantiomères de formule I
dans laquelle
R¹, R² désignent méthyle,
R³, R⁴ désignent H,
R⁵ désigne H,
R⁶ désigne méthyle,
sont séparés.

9. Méthode selon les revendications 1 à 8, **caractérisée en ce que** les énantiomères de formule I
dans laquelle
R¹, R² désignent méthyle,
R³, R⁴ désignent H,
R⁵ désigne H,
R⁶ désigne méthyle,
sont séparés.

10. Méthode selon les revendications 1 à 9, **caractérisée en ce que** le matériau d'échange d'ions comprend un sélecteur chiral, qui est composé d'un composant chiral et d'au moins un groupement d'échange d'ions, d'un espaceur et d'un support.

11. Méthode selon les revendications 1 à 10, **caractérisée en ce que** le composant chiral possède un poids moléculaire inférieur à 1000, et le groupement d'échange de cations est un groupement acide ayant un pKa < 4,0.

12. Méthode selon la revendication 11, **caractérisée en ce que** le groupement acide est un groupement d'acide carboxylique, sulfonique, sulfinique, phosphorique, phosphonique ou phosphinique.

13. Méthode selon les revendications 1-12, **caractérisée en ce qu'**un éluant comprenant
i) un solvant organique issu du groupe : méthanol, éthanol, propanol, acétonitrile, THF, dioxane, acétate d'éthyle, chloroforme, dichlorométhane, méthyl-tertio-butyléther, hexane, heptane, ou un mélange binaire, ternaire, quaternaire de ces solvants avec l'addition de co- et contre-ions ou également sans additifs ionogènes,
ii) un milieu aqueux avec ou sans addition de tampons et avec ou sans solvant organique polaire miscible issu du groupe tel que spécifié dans i.),
iii) du CO₂ supercritique ou subcritique avec ou sans solvant organique tel que spécifié dans i.), avec addition de co- et contre-ions ou également sans additifs ionogènes,
est utilisé.
